# EUROPEAN PATENT SPECIFICATION

(11) **EP 1 320 320 B1**
(45) Date of publication and mention of the grant of the patent: **29.09.2004**
(21) Application number: 01936806.7
(22) Date of filing: 18.05.2001
(51) Int. Cl.: A61B 3/10, A61B 3/11, A61F 9/013, A61F 9/01

(54) **APPARATUS FOR DETERMINING AND ABLATING THE CORNEAL TISSUE VOLUME NECESSARY FOR PERFORMING A CORNEAL LAMELLAR GRAFTING OPERATION**
VORRICHTUNG ZUR BESTIMMUNG UND ABTRAGUNG VON HORNHAUTGEWEBEVOLUMEN IN EINER LAMELLIERENDEN KERATOPLASTIK BEHANDLUNG
DISPOSITIF PERMETTANT, APRES DETERMINATION, DE PROCEDER A L'ABLATION DU VOLUME DE TISSU CORNEEN REQUIS EN VUE D'UNE GREFFE LAMELLAIRE DE LA CORNEE

(30) Priority: 15.09.2000 IT MI20002024
(43) Date of publication of application: 25.06.2003
(60) Divisional application: 04003911.7
(73) Proprietor: Ligi Tecnologie Medicali S.p.A., 74100 Taranto (IT)
(72) Inventor: D'IPPOLITO, Giuseppe, I-74100 Taranto (IT)
(74) Representative: Jorio, Paolo
(86) International application number: PCT/IT2001/000248
(87) International publication number: WO 2002/022003

(56) References cited:
- EP-A- 0 247 260
- EP-A- 0 255 581
- WO-A-00/27273
- WO-A-94/09849
- US-A- 5 963 300
- ECKHARDT HB ET AL.: "Lamellierende Keratoplastik mit dem Excimerlaser" DER OPHTHALMOLOGE, vol. 93, no. 3, 1996, pages 242-246, XP001018490

## Description

### BACKGROUND OF THE INVENTION

The present invention relates to an apparatus for determining and ablating the corneal tissue volume necessary for performing a corneal lamellar grafting operation, as optimized for each individual patient.

As is known, in a lamellar corneal grafting operation, a portion of corneal tissue having an even thickness and a variable diameter, depending on the amount and location of the different specific pathologies, is conventionally removed.

Usually, the corneal tissue is removed by a surgical instrument called "Krumeich's microkeratome" which, by a planing type of operation removes a corneal disc having a preset diameter and an approximatively even thickness. Laser ablation devices for performing a corneal grafting operation are described in WO94/09849 and in Eckkardt H.B. et al, Ophtalmologue (1996) 93:242 - 246.

### SUMMARY OF THE INVENTION

Thus, the aim of the present invention is to provide such an apparatus which is suitable to mutually coordinate an assembly of apparatus and which is specifically designed for defining, in an unique and optimum manner, the position, area and volume of the corneal tissue to be removed by a laser ablating operation, in order to optimally perform the ablating operation itself.

Within the scope of the above mentioned aim, a main object of the present invention is to provide such an apparatus which is very efficient in operation and which provides accurate values to allow the operator to perform optimally the ablating operation, i.e. in a manner optimized for each individual patient.

Yet another object of the present invention is to provide such an apparatus which, owing to its peculiar constructional features, is very reliable and safe in operation.

Yet another object of the present invention is to provide such an apparatus which can be easily made by using easily available elements and materials.

According to one aspect of the present invention, the above mentioned aim and objects, as well as yet other objects, which will become more apparent hereinafter, are achieved by an apparatus for determining and removing, by an ablating operation, a corneal tissue volume necessary for performing a lamellar corneal grafting operation, as optimized for each individual patient, characterized in that said apparatus comprises a central processing unit, to which are operatively connected a corneal pachymeter, a pupillometer and a photoablative laser as defined in the appended claims.

### BRIEF DESCRIPTION OF THE DRAWINGS

Further characteristics and advantages of the present invention will become more apparent hereinafter from the following disclosure of a preferred, though not exclusive, embodiment of an apparatus for determining and ablating an optimum volume of a corneal tissue as necessary for performing a lamellar corneal grafting operation, which is illustrated, by way of an indicative, but not limitative, example, in the accompanying drawings, where:
Figure 1 is a flow diagram illustrating the operating flows which can be performed by the apparatus according to the present invention;
Figure 2 is a cross-sectional view illustrating an optimum patient receiving bed;
Figure 2a is a further cross sectional view illustrating a typical section of a donor lens;
Figure 3 illustrates a typical section of the optimum receiving bed;
Figure 3a represents an optimum typical section of the donor lens;
Figure 4 illustrates a typical section of the optimum patient ablating contour, according to the present invention;
Figure 4a represents a typical section of the donor lens ablating contour; and
Figure 5 represents a block diagram of the ablating apparatus according to the present invention.

### DESCRIPTION OF THE PREFERRED EMBODIMENTS

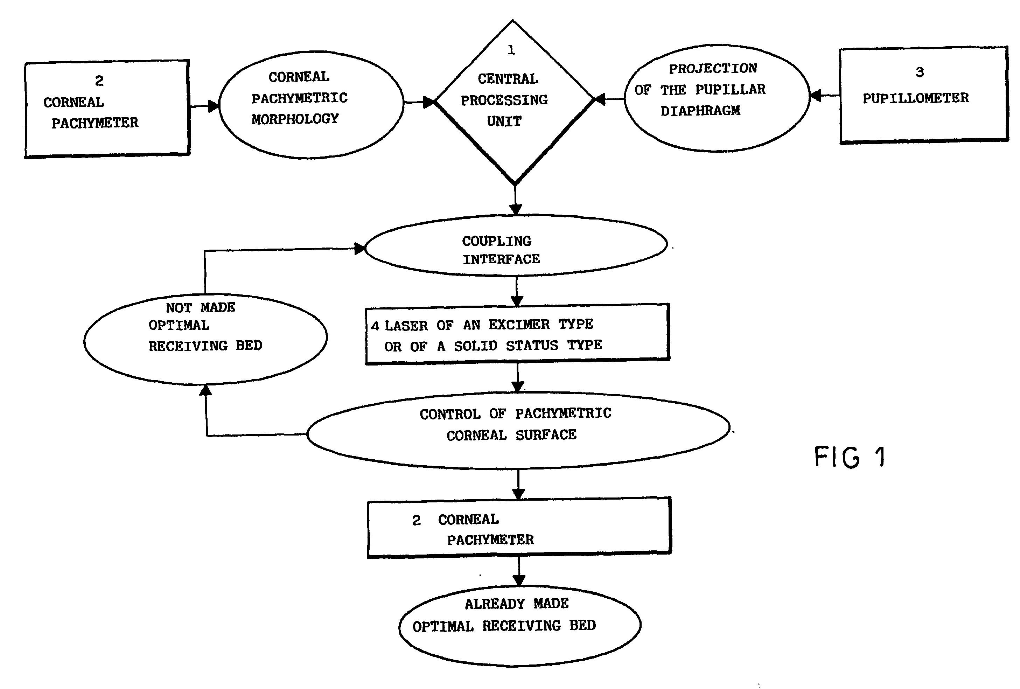

With reference to the number references of Figure 1, the apparatus for determining and removing, by an ablating operation, a corneal tissue volume necessary for a lamellar grafting transplantation, as optimized for the individual patient, comprises a central processing unit, generally indicated by the reference number 1, to which are coupled a corneal pachymeter (of an optical, ultrasound or the like type), generally indicated by the reference number 2, provided for morphologically detecting the corneal thickness and for tridimensionally mapping it.

To said central processing unit (1) a pupillometer, indicated by the reference number 3, for determining the projection of the pupillar diaphragm, at the level of the corneal front surface, is connected.

To said central processing unit 1 is moreover connected a photoablative laser (of an excimer, solid status, femtosecond or the like type), of a microspot type, generally indicated by the reference number 4, provided with a coupling interface, for reading the altimetric ablative datum, as expressed by microns on a x, y plane matrix or array.

The apparatus according to the present invention allows to find the ablating volume, which is obtained from a difference of the pachymetric map, as detected for the individual patient by the pachymeter 2, and the optimum pachymetric map of the bed receiving the donor lens.

The operator, in using the apparatus according to the invention, will detect at the start, by using the pupillometer 3, the projection of the pupil on the cornea front surface, the diameter thereof and the position of the related centroid with respect to the corneal limbus, as well as the diameter of the overall cornea and the position of its centroid, with respect to the pupillar centroid.

Then, the operator will detect the pachymetric data by using a corneal pachymeter (of an optical, ultrasonic or the like type) 2.

As is known, the pachymetric map is the tridimensional contour or profile which is best interpolatedly approximated to the data detected by the corneal pachymeter 2.

The optimum contour or profile of the donor lens receiving bed is defined by the operator by finding or detecting the following parameters:
- the center of the receiving bed;
- the diameter of the receiving bed;
- the minimum thickness at the center of the receiving bed
- the maximum thickness at the edge of the receiving bed; and
- the thickness variation along the diameter of the receiving bed.

The receiving bed center can be either selected between the corneal centroid, the projection on the cornea of the pupillar centroid, or arbitrarily by the operator.

The diameter of the receiving bed, in turn, is defined by the operator depending on the amount and location of the patient pathology.

The minimum and maximum thicknesses of the receiving bed are defined by the operator depending on the detected pathology analysis and the desired post-surgical refracting geometry.

The variation of the thickness along the receiving bed diameter is, depending on the operator selection, an even variation, a linear or exponential variation, depending on the desired post-surgical refracting geometry.

By using the definition of the above mentioned parameters, the operator will detect, in an unique and optimum manner for the patient, the receiving bed provided for receiving lens of the donor.

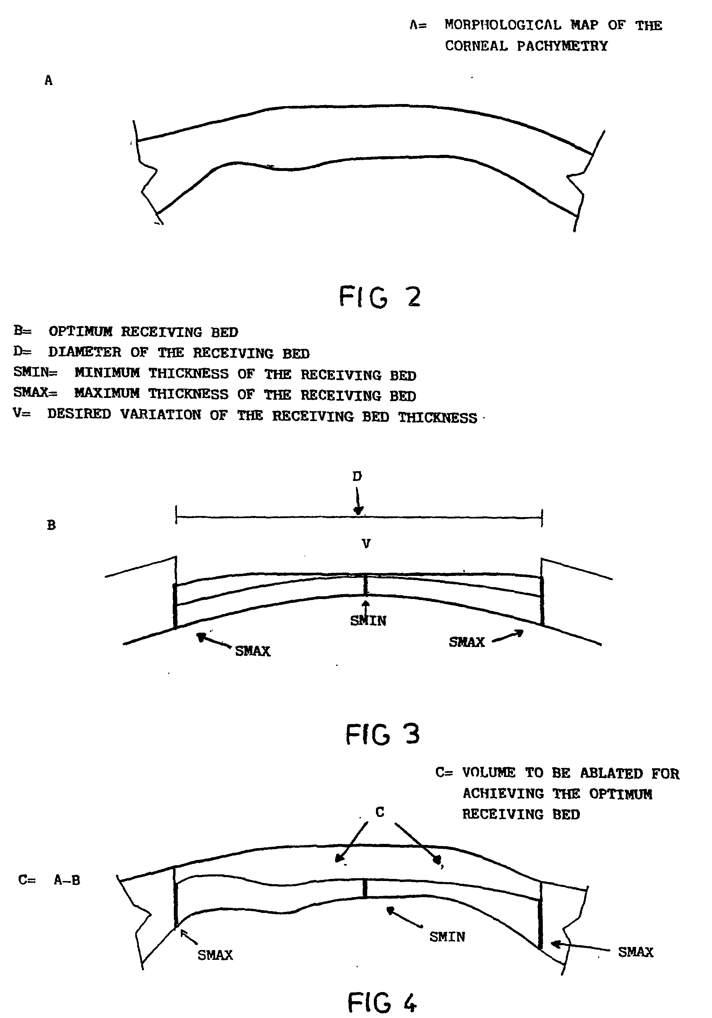

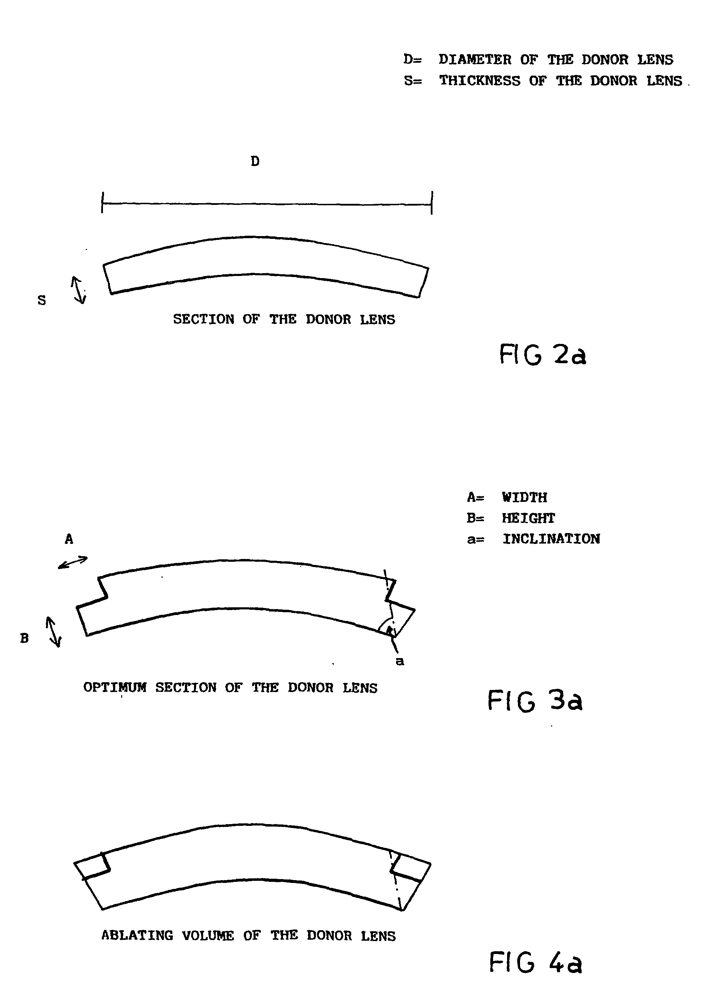

The ablating volume shown in Figure 2 is then obtained from the difference of the pachymetric map, as determined as above indicated, and the thus defined receiving bed.

The above disclosed method will univocally define the tissue to be ablated volume, in order to set an optimum patient receiving bed.

It is graphically represented by different color areas, clearly showing the receiving bed, its location and the residual cornea area not involved in the operation.

Moreover, digital data will express the overall ablating surface, the overall ablating volume, the maximum and minimum ablating volumes and related planimetric locations as well as the planimetric location of the ablating center with respect to the pupillar centroid.

The ablating volume related to the donor lens is obtained by defining the width A and height B of figure 3a to be generated on the lens or, alternately, the desired inclination or slope a.

The altimetric ablative datum is represented on a x, y-plane square matrix, to allow the photoablative laser 4 to properly perform its detection operation, through a suitable interface, for detecting the ablating contour or profile which, upon detection, is practically followed.

The system, moreover, optionally through an intra-operating detection of the pachymetric datum, will verify that the treatment has been carried out according to the programmed ablating strategy, while modifying the number of surface-unit localized pulses.

In this case, the operation will end upon achieving a congruency of the detected and desired data.

It should be pointed out that figure 3 clearly shows the optimum receiving bed B, the letter D showing the diameter of said receiving bed.

In this figure, Smin shows the minimum thickness of the receiving bed, whereas Smax shows the maximum thickness of said receiving bed.

The letter V shows, in turn, the desired variation of the receiving bed thickness.

In figure 4, the letter C shows the volume to be ablated for achieving the optimum receiving bed.

In this figure, moreover, Smax and Smin show respectively the maximum and minimum thickness of the mentioned receiving bed.

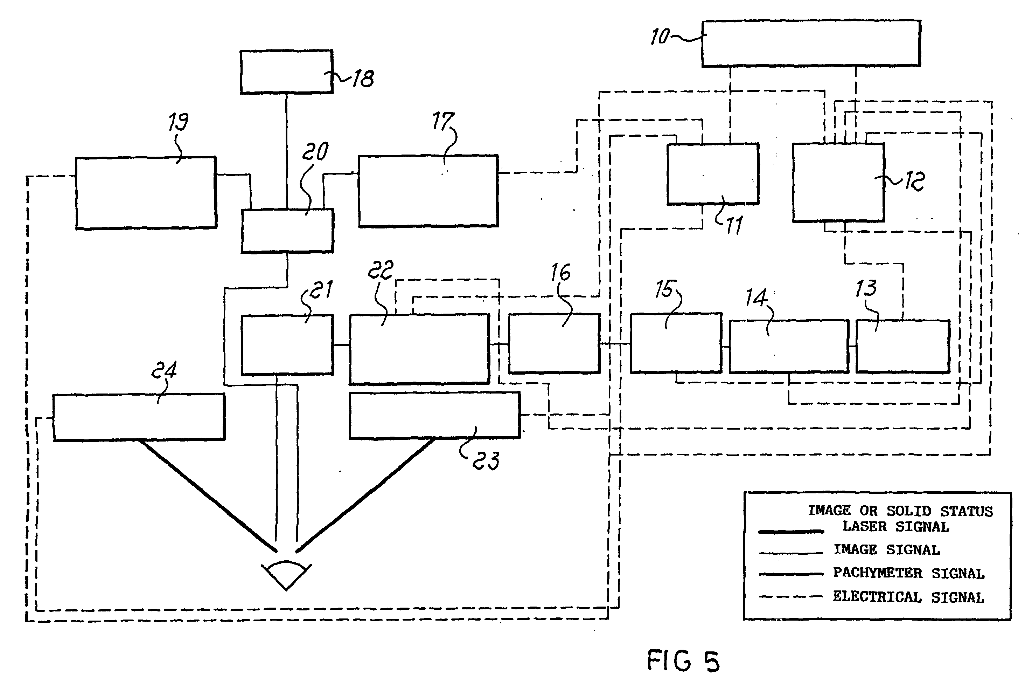

With reference to figure 5, which is a block diagram of the apparatus according to the invention, said apparatus comprises a central processing unit 10, which is coupled to a pachymetric control unit 11 and a photoablative laser control unit 12.

The latter is coupled to a laser cavity 13, fitted, in turn, to a control device for controlling the power of the laser beam 14 and to a device 15 for measuring the laser beam power.

This apparatus is moreover characterized in that it further comprises a focalizing system 16 for focalizing the laser beam, coupled to a galvanometric system 22 and to an orienting system 21 for orienting the laser beam.

Moreover, a system 23 for detecting the corneal pachymetry and a source 24 for detecting said corneal pachymetry are moreover provided.

The apparatus comprises furthermore an optic divider 20, to divide or split the video signal between an operating microscope 18, a video camera 19 for detecting the ocular motility, coupled to the photoablative laser control unit 12 and a further video camera 17 for detecting the ocular motility, coupled to the pachymeter control unit 11.

From the above disclosure it should be apparent that the invention fully achieves the intended aim and objects.

In particular, an apparatus according to the block diagram shown in figure 5 has been provided, which is adapted to allow to detect in a very accurate manner the data and/or parameters necessary for addressing and properly performing the surgical operation.

The invention, as disclosed, is susceptible to several modifications and variations, all of which will come within the scope of the invention defined in the appended claims.

Moreover, the used materials, as well as the contingent size and shapes, can be any, depending on requirements.

## Claims

1. An apparatus for determining and removing, by an ablating operation, a corneal tissue volume necessary for performing a lamellar corneal grafting operation, as optimized for each individual patient, comprising a central processing unit, to which are operatively connected a corneal pachymeter, a pupillometer and a microspot photoablative laser controlled by a microspot photoablative laser control unit, **characterized in that** said corneal pachymeter is designed for performing a morphologic detection and a tridimensional mapping of the cornea thickness, said pupillometer is designed for determining the projection of the pupillar diaphragm at the level of the cornea front surface, the diameter thereof and the location of the centroid thereof, with respect to a corneal limbus, and the diameter of the overall cornea and the location of the centroid thereof with respect to the pupillar centroid, and that said microspot ablative laser comprises a laser cavity to which said microspot ablative laser control unit is coupled, said cavity being in turn coupled to a control device for controlling the power of the microspot laser beam and to a device for measuring the power of the microspot laser beam.

2. An apparatus according to Claim 1, **characterized in that** said microspot photoablative laser is coupled to said central processing unit through an interface and is controlled by said central processing unit so as to read an altimetric ablating datum expressed in microns on a x, y-plane cartesian matrix.

3. An apparatus according to Claims 1 and 2, **characterized in that** said ablation volume is obtained from a difference of a pachymetric map detected for an individual patient by said pachymeter and an optimum pachymetric map of the eye lens receiving bed.

4. An apparatus according to Claims 1 to 3, **characterized in that** said central processing unit determines an optimum contour of the donor eye lens receiving bed by processing a series of parameters including the center of said receiving bed, the diameter of said receiving bed, the minimum thickness at the center of said receiving bed, the maximum thickness at the edge of said receiving bed and a thickness variation along the diameter of said receiving bed.

5. An apparatus according to Claim 4, **characterized in that** said center of said receiving bed is either selected between the corneal centroid, the projection of the pupillar centroid on the cornea or arbitrarily by the operator.

6. An apparatus according to Claim 4, **characterized in that** said diameter of said receiving bed is determined depending on the amount and location of the patient detected pathology.

7. An apparatus according to Claim 1, **characterized in that** said central processing unit provides digital data indicating the overall ablating surface, the overall ablating volume, the ablating maximum and minimum thicknesses and related planimetric offset or dislocation, and the planimetric offset or dislocation of the ablating center from the pupillar centroid.

8. An apparatus according to Claim 1, **characterized in that** said apparatus further comprises a focalizing system for focalizing said laser beam, said focalizing system being coupled to an orienting galvanometric system for orienting the laser beam and to a laser beam orienting mirror system.

9. An apparatus according to Claim 1, **characterized in that** said apparatus further comprises an operating microscope coupled to an optic divider, in turn connected to a video camera for detecting the ocular motility.

10. An apparatus according to Claims 1 and 9, **characterized in that** said apparatus comprises a pachymetric control unit coupled to said video camera for detecting said ocular motility.

11. An apparatus according to Claim 1, **characterized in that** said apparatus further comprises an orienting mirror system, a corneal pachymetry detecting system and a corneal pachymetry detecting source.

## Patentansprüche

1. Vorrichtung zum Bestimmen und Entfernen, durch einen Abtragungsvorgang, eines Hornhautgewebevolumens, das zum Durchführen einer lamellären Keratoplastikbehandlung, optimiert für jeden einzelnen Patienten, notwendig ist, mit einer Zentraleinheit, mit welcher ein Hornhautdickenmessgerät, ein Pupiliometer und ein durch eine photoablative Mikropunktlaser - Steuereinheit gesteuerter photoablativer Mikropunktlaser wirkverbunden sind,
**dadurch gekennzeichnet,**
**dass** das Hornhautdickenmessgerät zum Durchführen einer morphologischen Erfassung und einer dreidimensionalen Abbildung der Hornhautdicke ausgebildet ist,
**dass** das Pupillometer zum Bestimmen der Projektion der Pupillenmembran auf der Ebene der vorderen Hornhautoberfläche, deren Durchmesser und der Position deren Schwerpunkts in Bezug auf einen Perikornealring, sowie des Durchmessers der gesamten Hornhaut und der Position deren Schwerpunkt in Bezug auf den Pupillenschwerpunkt ausgebildet ist, und
**dass** der photoablative Mikropunktlaser einen Laserresonator aufweist, mit welchem die photoablative Mikropunktlaser - Steuereinheit gekoppeit ist, wobei der Resonator wiederum mit einem Steuergerät zum Steuern der Energie des Mikropunktiaserstrahls und mit einem Gerät zum Messen der Energie des Mikropunktlaserstrahls gekoppelt ist.

2. Vorrichtung nach Anspruch 1,
**dadurch gekennzeichnet,**
**dass** der photoablative Mikropunktlaser mit der Zentraleinheit durch eine Schnittstelle verbunden ist und durch die Zentraleinheit so gesteuert wird, dass er eine altimetrische Abtragungsbezugsgröße, ausgedrückt in Mikrometer, auf einer kartesischen x-y-Matrixebene liest.

3. Vorrichtung nach Ansprüchen 1 und 2,
**dadurch gekennzeichnet,**
**dass** man das Abtragungsvolumen aus einem Unterschied einer für einen einzelnen Patienten durch das Dickenmessgerät erfassten Dickenmessabbildung und einer optimalen Diokenmessabbildung des Augenlinsen-Aufnahmebetts erhält.

4. Vorrichtung nach Ansprüchen 1 bis 3,
**dadurch gekennzeichnet,**
**dass** die Zentraleinheit einen optimalen Umriss des Augenlinsen-Aufnahmebetts des Spenders durch Verarbeiten einer Reihe von Parametern, einschließlich des Zentrums des Aufnahmebetts, des Durchmessers des Aufnahmebetts, der minimalen Dicke im Zentrum des Aufnahmebetts, der maximalen Dicke am Rand des Aufnahmebetts und einer Dickenänderung entlang des Durchmessers des Aufnahmebetts, bestimmt.

5. Vorrichtung nach Anspruch 4,
**dadurch gekennzeichnet,**
**dass** das Zentrum des Aufnahmebetts entweder zwischen dem Hornhautschwerpunkt, der Projektion des Pupillenschwerpunkts auf der Hornhaut oder willkürlich durch den Bediener ausgewählt wird.

6. Vorrichtung nach Anspruch 4,
**dadurch gekennzeichnet,**
**dass** der Durchmesser des Aufnahmebetts in Abhängigkeit von dem Ausmaß und der Position der am Patienten erfassten Krankheit bestimmt wird.

7. Vorrichtung nach Anspruch 1,
**dadurch gekennzeichnet,**
**dass** die Zentraleinheit digitale Daten bereitstellt, die die gesamte Abtragungsfläche, das gesamte Abtragungsvolumen, die maximale und die minimale Abtragungsdicke und eine zugehörige planimetrische Versetzung oder Verschiebung sowie die planimetrische Versetzung oder Verschiebung des Abtragungszentrums von dem Pupillenschwerpunkt angeben.

8. Vorrichtung nach Anspruch 1,
**dadurch gekennzeichnet,**
**dass** die Vorrichtung ferner ein Fokussiersystem zum Fokussieren des Laserstrahls aufweist, wobei das Fokussiersystem mit einem galvanometrischen Orientierungssystem zum Orientieren des Laserstrahls und mit einem Laserstrahl-Orientierungsspisgelsystem gekoppelt ist.

9. Vorrichtung nach Anspruch 1,
**dadurch gekennzeichnet,**
**dass** die Vorrichtung ferner ein Betriebsmikroskop aufweist, das mit einem optischen Teiler gekoppelt ist, der wiederum mit einer Videokamera zum Erfassen der Okularmotilität verbunden ist.

10. Vorrichtung nach Ansprüchen 1 und 9,
**dadurch gekennzeichnet,**
**dass** die Vorrichtung eine Dickenmessungs-Steuereinheit aufweist, die mit der Videokamera zum Erfassen der Okularmotilität gekoppelt ist.

11. Vorrichtung nach Anspruch 1,
**dadurch gekennzeichnet,**
**dass** die Vorrichtung ferner ein Orientierungsspiegelsystem, ein Hornhautdickenmesserfassungssystem und eine Hornhautdickenmesserfassungsquelle aufweist.

## Revendications

1. Un appareillage pour la détermination et l'élimination, par une opération d'ablation, du volume de tissu cornéen nécessaire pour procéder à une opération de greffage cornéen lamellaire optimisée pour chaque patient individuel, appareillage comprenant une unité centrale de traitement à laquelle sont connectés fonctionnellement un pachymètre cornéen, un pupillomètre et un laser photoablatif à microspot contrôlé par une unité de contrôle de laser photoablatif à microspot,
**caractérisé en ce que**
ledit pachymètre cornéen est connu et agencé de manière à procéder à une détection morphologique et à une cartographie tridimensionnelle de l'épaisseur de la cornée,
ledit pupillomètre est connu et agencé de manière à déterminé la projection du diaphragme pupillaire au niveau de la surface frontale cornéenne, dont le diamètre et l'emplacement du centroïde, par rapport à un limbe cornéen et le diamètre de la cornée totale et l'emplacement de son centroïde par rapport au centroïde pupillaire
et **en ce que** ledit laser ablatif à microspot comprend une cavité de laser à laquelle ladite unité de contrôle de laser ablatif à microspot est couplée, ladite cavité étant à son tour couplée à un dispositif de contrôle destiné à contrôler l'énergie du faisceau laser à microspot et à un dispositif de mesure de l'énergie du faisceau laser à microspot.

2. Un appareillage selon la revendication 1, **caractérisé en ce que** ledit laser photoablatif à microspot est couplé à ladite unité centrale de traitement par une interface et est contrôlé par ladite unité centrale de traitement de manière à lire une valeur altimétrique d'ablation exprimée en microns sur une matrice cartésienne dans un plan x-y.

3. Un appareillage selon les revendications 1 et 2, **caractérisé en ce que** ledit volume d'ablation est obtenu par différence entre une carte pachymétrique détectée pour un patient individuel par ledit pachymètre et une carte pachymétrique optimale du lit recevant le cristallin de l'oeil, ou iris.

4. Un appareillage selon la revendications 1 à 3, **caractérisé en ce que** ladite unité centrale de traitement détermine un contour optimal du iris du cristallin du donneur en traitant une série de paramètres regroupant le centre dudit iris, le diamètre dudit iris, l'épaisseur minimale au centre dudit iris, l'épaisseur maximale au bord dudit iris et une variation de l'épaisseur le long du diamètre dudit iris.

5. Un appareillage selon la revendication 4, **caractérisé en ce que** ledit centre dudit iris est soit choisi entre le centroïde cornéen et la projection du centroïde pupillaire sur la cornée, soit choisi arbitrairement par l'opérateur.

6. Un appareillage selon la revendication 4, **caractérisé en ce que** ledit diamètre dudit iris est déterminé en fonction de l'importance et de l'emplacement de la pathologie détectée chez le patient.

7. Un appareillage selon la revendication 1, **caractérisé en ce que** ladite unité centrale de traitement fournit des données digitales indiquant la surface totale de l'ablation, le volume total de l'ablation, les épaisseurs maximale et minimale de l'ablation et le décalage ou déplacement planimétrique associé, ainsi que le décalage ou déplacement du centre de l'ablation par rapport au centroïde pupillaire.

8. Un appareillage selon la revendication 1, **caractérisé en ce que** ledit appareillage comprend au surplus un système de focalisation destiné à focaliser ledit faisceau laser, ledit système de focalisation étant couplé à un système d'orientation galvanométrique en vue d'orienter ledit faisceau laser et à un système à miroir d'orientation du faisceau laser.

9. Un appareillage selon la revendication 1, **caractérisé en ce que** ledit appareillage comprend au surplus un microscope opératoire couplé à un diviseur optique, lui-même connecté à une caméra vidéo en vue de détecter la motilité oculaire.

10. Un appareillage selon les revendications 1 et 9, **caractérisé en ce que** ledit appareillage comprend une unité de contrôle pachymétrique couplée à ladite caméra vidéo en vue de détecter ladite motilité oculaire.

11. Un appareillage selon la revendication 1, **caractérisé en ce que** ledit appareillage comprend au surplus un système d'orientation à miroir, un système de détection cornéenne pachymétrique et une source de détection cornéenne pachymétrique.
